Europäisches Patentamt

European Patent Office

Office européen des brevets

(19)

(11) Publication number: 0 375 976

A2

(12) **EUROPEAN PATENT APPLICATION**

(21) Application number: 89122080.8

(22) Date of filing: 30.11.89

(51) Int. Cl.5: **A61K 31/725, A61K 31/795, A61K 31/00**

The title of the invention has been amended (Guidelines for Examination in the EPO, A-III, 7.3).

(30) Priority: 01.12.88 IL 88554

(43) Date of publication of application:
**04.07.90 Bulletin 90/27**

(84) Designated Contracting States:
**DE ES FR GB IT NL SE**

(71) Applicant: **Hadassa Medical Organization**
**P.O. Box 12000**
**Jerusalem(IL)**

Applicant: **YEDA RESEARCH AND**
**DEVELOPMENT COMPANY LIMITED**
**P.O. Box 95**
**Rehovot 76100(IL)**

(72) Inventor: **Cohen, Irun R.**
**11 Hankin Street**
**Rehovot(IL)**
Inventor: **Miller, Theodore D.**
**10 Twickenham Court**
**Thornhill Ontario L3T 5T7(CA)**
Inventor: **Naparstek, Yaacov**
**17 Davidson Street**
**Jerusalem(IL)**

(74) Representative: **Vossius & Partner**
**Siebertstrasse 4 P.O. Box 86 07 67**
**D-8000 München 86(DE)**

(54) **Compounds binding to a heparin receptor as inhibitors of T cell-mediated immune reactions.**

(57) There are provided compositions for the inhibition of T-Cell-mediated immune reactions, which comprise an effective quantity of Fragmin (T.M.) of polyanethol sulfonate, of dextran sulfate or of another compound which binds to the heparin receptor. The compositions of the invention are effective as active ingredient of pharmaceutical compositions for the treatment of patients afflicted with metastatic tumor cells. Another feature of the invention is an assay for the determination of inhibitory effects of such compositions on T-cell immunity in vivo by their ability to compete with heparin for binding in the heparin receptor.

EP 0 375 976 A2

## Compositions containing a compound binding to a heparin receptor

The present invention relates to compositions for the inhibition of T cell-mediated immune reactions which contain an effective quantity of a compound binding to the heparin receptor. In a preferred embodiment of the present invention said compositions are pharmaceutical compositions.

As described in EP-A-0 251 134 heparins may be used to inhibit T lymphocyte movement into tissues and autoimmune reactions or graft rejection by virtue of their inhibition of the T lymphocyte enzyme heparanase.

The present invention is based on new information regarding the mechanism of heparanase inhibition involving the discovery of a cell membrane receptor for heparin.

The invention demonstrates that heparin-like effects of other molecules on heparanase can be predicted by an assay based on competition with heparin for binding to the specific membrane receptor. Furthermore, it is demonstrated that polyenatholsulfonate or dextran sulfate compete with heparin for binding and also inhibit immune reactivity, as two specific examples.

It is apparent that inhibition of delayed type hypersensitivity reactions is associated with inhibition of the synthesis and expression of heparanase.

The embodiments of the present invention are characterized in the claims.

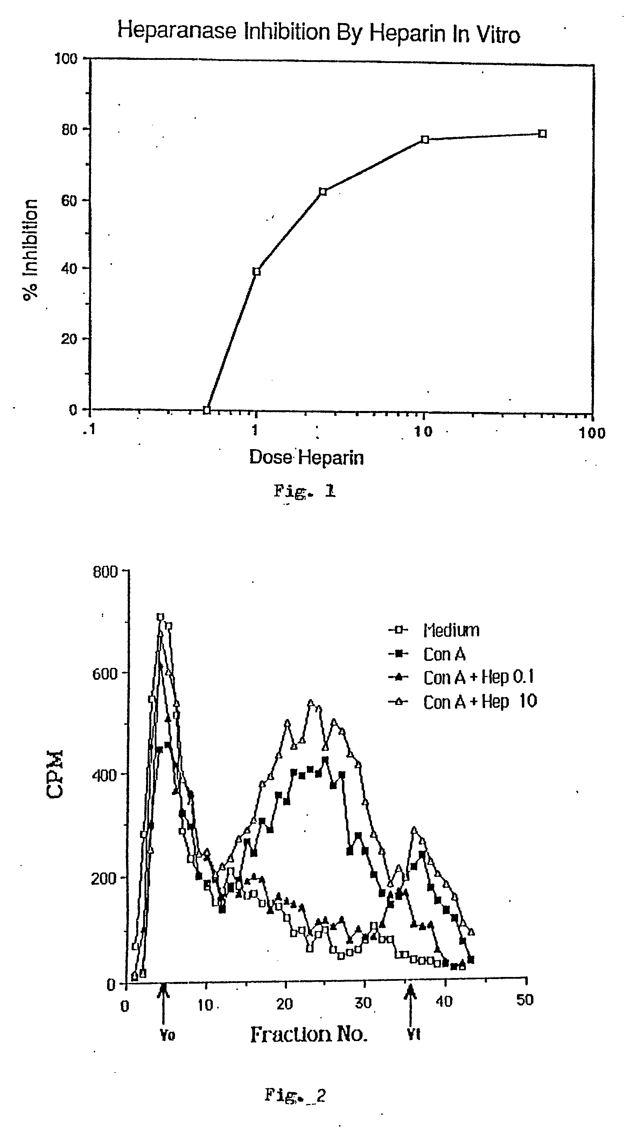

Figure 1 demonstrates the dose-response effects of heparin in competition with free heparanase. It can be seen that increasing amounts of heparin progressively inhibit free heparanase until a plateau is reached at about 10 $\mu$g/ml of heparin. There is no decrease in inhibition at high concentrations of heparin.

Figure 2 illustrates the effects of two doses of heparin (0.1 ug/ml and 10 $\mu$/ml) on the heparanase activity of lymphocytes in vitro. The concentration of 0.1 $\mu$g/ml caused inhibition of enzyme activity, while the higher concentration of 10 $\mu$g/ml of heparin did not inhibit the enzyme.

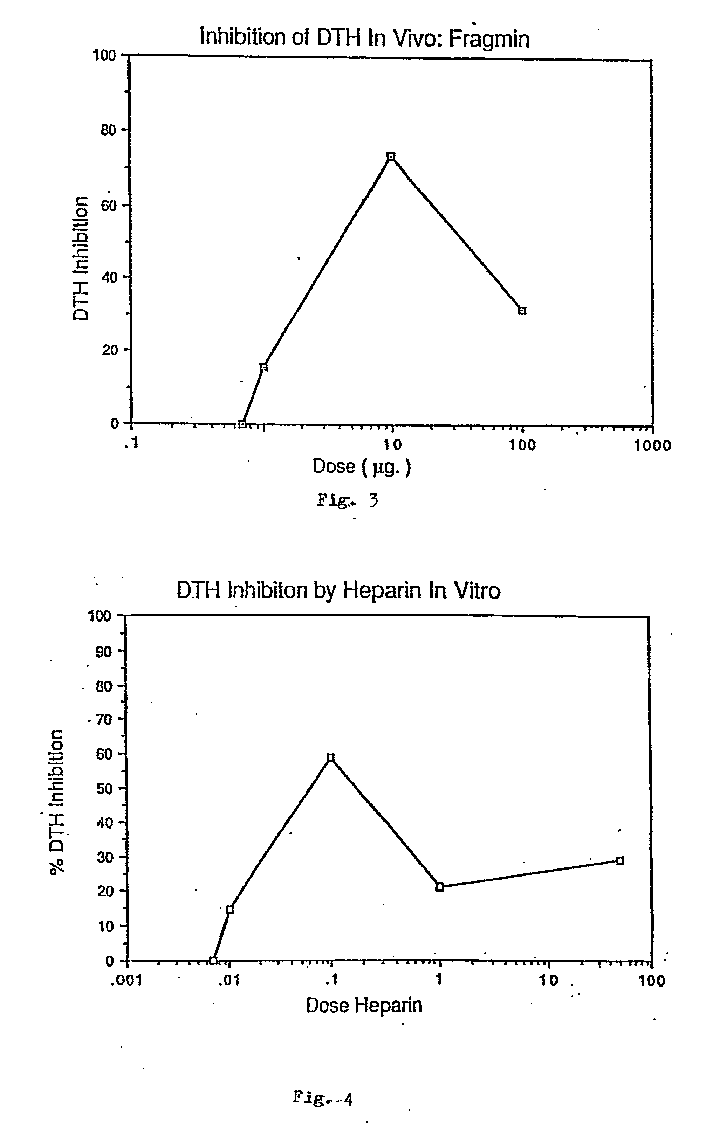

Figure 3 illustrates the inhibition of delayed type hypersensitivity by heparin administered in vivo. Here too the higher concentration (100 $\mu$g/rat/day)was much less effective than the optimal dose of heparin (10 ug/rat/day).

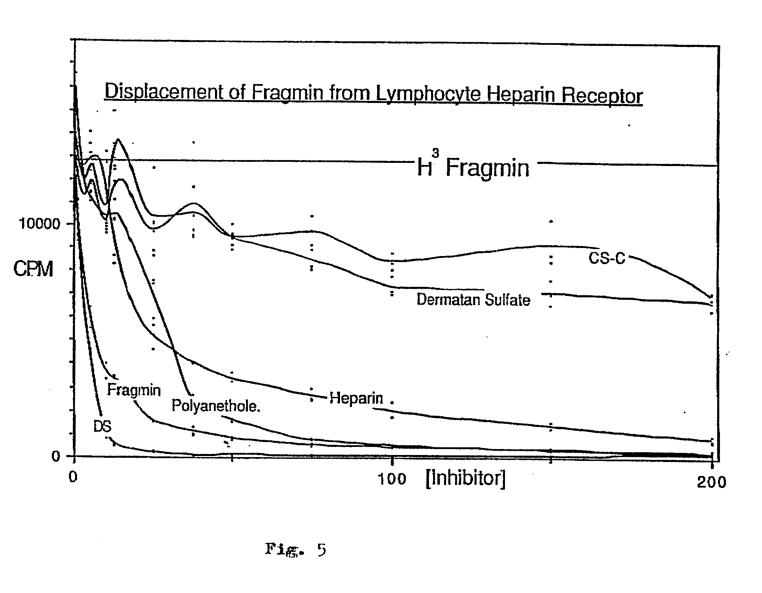

Figure 4 shows that treatment of lymphocytes in vitro with heparin inhibits their ability to mediate a delayed hypersensitivity reaction with a dose-response inhibition curve showing the same loss of effect at higher than optimal doses of heparin, 1 $\mu$g/ml and 50 $\mu$g/ml were less inhibitory than 0.1 $\mu$g/ml.

These results indicate that heparin inhibits cell-mediated immunity by inhibiting lymphocyte synthesis and/or expression of heparanase with a dose response curve that is not consistent with direct competitive inhibition of the heparanase enzyme. Direct enzyme inhibition does not show a loss of effect at higher than optimal concentrations,while the inhibition of enzyme expression does.

The dose-response effects of heparin in inhibiting lymphocyte heparanase suggested that heparin interacts with a receptor at the cell surface.

Figure 5 shows evidence for a specific receptor. Displacement of ($H^3$) fragmin, a low molecular weight heparin, was used to detect the interaction of various molecules with a putative heparin receptor. It can be seen that unlabeled fragmin and heparin were good inhibitors. Chondroitin sulfate-C and dermatan sulfate were not good inhibitors of heparin binding, although these molecules, similar to heparin, are polysulfated polysaccharides. This indicates the presence of a specific receptor on the lymphocyte surface. It is thus apparent that heparin interacts with lymphocytes via a membrane receptor. Furthermore, we have demonstrated that polyanethol-sulfonate binds to heparin receptor in vitro and inhibits cell-mediated immunity in vivo.

Figure 5 shows that the molecule polyanethol sulfonate also interacts with the heparin receptor. This suggests that, like heparin, it might also serve to inhibit T cell-mediated reactivity. This was measured by treating mice subcutaneously with polyanethol sulfonate (PASA), 10 $\mu$g/mouse/day, and measuring the effect on mediation of delayed type hypersensitivity to the skin sensitizing chemical DNFB.

Figure 6 shows that polyanethol sulfonate, like heparin (Diosynth), inhibited the response. CS-C which did not bind to the heparin receptor (see Figure 5), did not inhibit the DTH.

Thus, the inhibitory effect of a drug could be predicted by its capacity to compete with heparin for binding to the heparin receptor.

Furthermore, the results demonstrate that dextran sulfate binds to heparin receptor in vitro and inhibits cell-mediated immunity in vivo.

Figure 5 shows that dextran sulfate (molecular weight 5000; or DS), similar to polyanethol-sulfonate, inhibited the binding of heparin to the heparin receptor. This suggests that, like heparin, dextran sulfate is likely to inhibit T cell-mediated reactions. This was verified by treating groups of mice with a daily subcutaneous inoculation of dextran sulfate at doses of 1, 10, 100 or 500 $\mu$g.

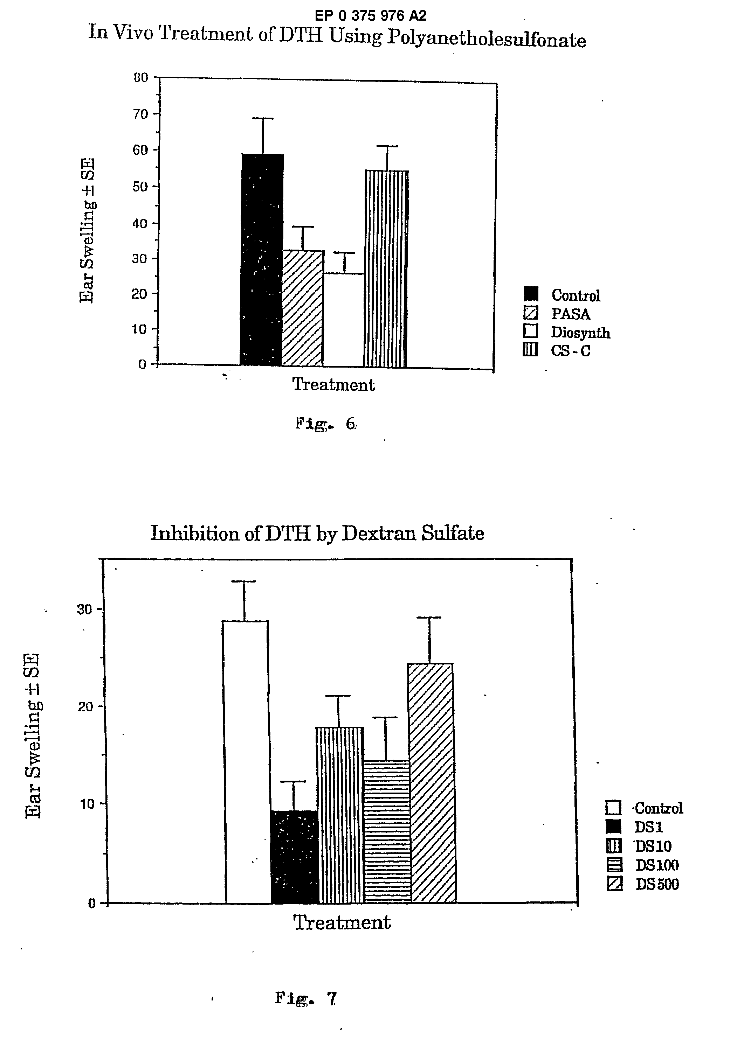

Figure 7 shows that, like heparin, a relatively low dose of dextran sulfate (1 $\mu$g/daily) markedly inhibits a DTH reaction to the sensitizing chemical DNFB. A higher dose of dextran sulfate (500

μg/daily) was not inhibitory. Thus the inhibitory effect of dextran sulfate on T cell immunity in vivo could be predicted by its ability to compete with heparin for binding to the heparin receptor.

The invention is illustrated with reference to the enclosed Figures, in which

Fig. 1 illustrates the inhibition of heparanase activity by heparin in vitro;

Fig. 2 illustrates the inhibition of lymphocyte expression of heparanase by heparin;

Fig. 3 illustrates the inhibition of DTH in vivo;

Fig. 4 illustrates the DTH inhibition by heparin in vitro;

Fig. 5 illustrates the displacement of Fragmin from lymphocyte heparin receptor;

Fig. 6 illustrates the effects of in vivo treatment of DTH using polyanethol sulfonate;

Fig. 7 illustrates the inhibition of DTH by dextran sulfate.

It is evident from the graph of Fig. 1 that heparin directly inhibits heparanase activity with no loss of activity at higher than optimal concentrations. Heparanase derived from ESb lymphoma cells was placed on $^{35}$S-ECM with various doses of heparin incubated under appropriate conditions (pH 6.25, 7.5% $CO_2$, 37°C) for 24 hours. The supernatant was then passed through a sepharose 6b column to yield a profile of enzymatic activity. The heparanase activity is equal to the area under the peak of radioactivity containing the low molecular weight degradation fragments of heparan sulfate.

(An example of the peak of low molecular weight fragments can be seen in Figure 2, between fractions 10 and 35. The high molecular weight undegraded heparan sulfate can be seen between fractions 0 and 10). The peak of activity without added heparin was compared to that obtained in the presence of concentrations of heparin of 0.5, 1, 3, 10 or 50 μg/ml and computed as a percent inhibition.

As illustrated with reference to Figure 2, heparin inhibits lymphocyte expression of heparanase with a loss of inhibitory activity at high concentration. Splenocytes of BALB/c mice (female 6-8 weeks) were stimulated using Concanavalin A for 48 hours in the presence of concentrations of heparin of 0.1 μg/ml or 10 μg/ml. The cells were then lysed and the cytoplasmic fraction was assayed for heparanase as indicated in Figure 1. Here the actual peaks are shown.

It is apparent from the graph of Figure 3, that fragmin inhibits DTH reactions in vivo with a loss of activity at a higher concentration. BALB/c mice were sensitized topically with 0.5% DNFD (Kodak), on their abdomens. Five days later Fragmin (KabiVitrum) was injected subcutaneously in the flanks at the indicated doses 18 and 1 hour previous to challenge with DNFB (0.2%) on the animal's ears. DTH was measured as a function of ear swelling in comparison to a group of mice that was sensitized to DNFB but which was not treated with heparin.

Figure 4 illustrates that heparin inhibits adoptive transfer of DTH by sensitized lymphocytes with a loss of inhibitory activity at higher concentrations. BALB/c mice were sensitized to DNFB (0.5%) topically on their abdomens and footpads. Four days later the animals were sacrificed and their axial and inguinal lymph nodes were pooled to produce a single cell suspension which was then treated for 48 hours with Concanavalin A (1 μg/5x10$^5$ cells/ml) and various doses of heparin. 50x10$^6$ activated lymphocytes were injected i.v. into each naive recipient mouse (age, sex, and strain-matched) and the transfer of the DTH was assayed as above. Inhibition of the DTH reaction was computed in comparison to that transferred by sensitized lymphocytes that had not been incubated with heparin.

Membranes of spleen cells contain a receptor specific for heparin (Fragmin). Various molecules similar in charge and/or shape to heparin were studied unlabelled for their ability to displace $^3$H-Fragmin from isolated splenocyte membranes. These materials included chondroitin sulfate B (CS-B), dermatan sulfate-C, Polyanethol-sulfonate (Polyanethol), 5000 molecular weight dextran sulfate (DS), fragmin and heparin. Results are shown in Figute 5. Membranes were incubated with the $^3$H-Fragmin and inhibitors for 2 hours in 200 μl DDW at 37-C. Samples were harvested and counted. Specific inhibition of Fragmin binding indicates a specific receptor.

Polyanethol-sulfonate treatment inhibits DTH. DTH was performed with reference to the experiments shown in Figure 3. Polyanethol-sulfonate (PASA) was assayed against heparin (Diosynth) or CS-C to measure its inhibitive effects. The results are illustrated with reference to Figure 6

As shown in the graph of Figure 7, dextran sulfate treatment inhibits DTH. DTH was performed as indicated in Figure 3. Dextran sulfate was assayed at various doses for inhibition of the DTH reaction to DNFB.

## Claims

1. A composition for the inhibition of T cell-mediated immune reactions, comprising an effective quantity of a compound binding to the heparin receptor.

2. A composition according to claim 1, wherein said compound is Fragmin.

3. A composition according to claim 1, wherein said compound is dextran sulfate.

4. A composition according to claim 1, wherein said compound is polyanethol sulfonate.

5. A composition according to claim 1, wherein said compound binds to the heparin receptor in competition with heparin and has a similar effect to heparin.

6. A composition according to any one of claims 1 to 5, which is a pharmaceutical composition.

7. A composition according to claim 6 for the treatment of patients afflicted with metastatic tumor cells.

8. A composition according to any one of claims 1 to 5, which is a diagnostic composition for the determination of heparin-like effects on immunity or heparanase by measuring the binding of said compound contained therein to the heparin receptor.

## Heparanase Inhibition By Heparin In Vitro

Fig. 1

Fig. 2

Inhibition of DTH In Vivo: Fragmin

Fig. 3

DTH Inhibiton by Heparin In Vitro

Fig.-4

**Displacement of Fragmin from Lymphocyte Heparin Receptor**

Fig. 5

## In Vivo Treatment of DTH Using Polyanetholesulfonate

Fig. 6.

## Inhibition of DTH by Dextran Sulfate

Fig. 7